# EUROPEAN PATENT SPECIFICATION

(11) **EP 2 453 864 B1**
(45) Date of publication and mention of the grant of the patent: **14.09.2016**
(21) Application number: 10800673.5
(22) Date of filing: 19.07.2010
(51) Int. Cl.: A61H 1/00, B05B 17/00, A61M 11/00, A61M 15/00

(54) **SYSTEMS AND METHODS FOR DRIVING SEALED NEBULIZERS**
NEGATIV VORGESPANNTE ABGEDICHTETE ZERSTÄUBER SOWIE SYSTEME UND VERFAHREN DAFÜR
SYSTÈMES ET MÉTHODES DE COMMANDE DE NÉBULISEURS ÉTANCHES

(30) Priority: 17.07.2009 US 226591 P
(43) Date of publication of application: 23.05.2012
(73) Proprietor: Nektar Therapeutics, San Carlos, CA 94070 (US)
(72) Inventor: GORDON, Benjamin Morris, Cambridge Cambridgeshire CB1 7RT (GB); GARDNER, Steven David, Yaxley Cambridgeshire PE7 3EX (GB); HAYES, Matthew James, Great Shelford Cambridge Cambridgeshire CB22 5LJ (GB)
(74) Representative: Henseler, Daniela
(86) International application number: PCT/US2010/042473
(87) International publication number: WO 2011/009133

(56) References cited:
- JP-A- H05 261 324
- US-A- 3 819 961
- US-A- 5 349 852
- US-A- 5 853 665
- US-A1- 2002 129 813
- US-A1- 2005 224 076
- US-A1- 2006 102 172
- US-A1- 2007 152 081
- US-B2- 6 569 109
- US-B2- 6 978 779
- US-B2- 7 155 980
- US-B2- 7 197 948

## Description

The invention relates to a method and a system for controlling the vibration of an element of a nebulizer according to the preambles of claims 1 and 10.

### BACKGROUND OF THE INVENTION

The features of the preambles of claims 1 and 10 are known from US 2005/0224076 A1. US 2005/0224076 A1 discloses an aerosol generation device comprising an oscillatable membrane for the nebulization of a liquid, having a first curved region and at least a second region which is surrounded by the first region, and wherein the first region and the second region have different curvatures. The oscillation of the membrane is thereby influenced such that less liquid adheres to the aerosol side of the membrane. Additional measures for preventing that liquid droplets adhere, such as the generation of a negative pressure in a liquid storing means, are not necessary.

US 3,819,961 A discloses an arrangement for generating ultrasonic oscillations, which may be rendered suitable for performing widely different kinds of operations such as welding, drilling cleaning.

US 2002/0129813 A1 teaches a method for aerosolizing a liquid comprising an aerosol generator having a plate with a plurality of apertures and a piezoelectric element to vibrate the plate. The energy level is adjusted to vibrate the plate at its instantaneous resonant frequency during aerosolization of the liquid.

JP H05 261324 A discloses a compact, light-weight and low cost ultrasonic misting device which can be driven using a low voltage power supply, with a superior power supply efficiency and a simply structured drive circuit.

US 6,569,109 B2 relates to an ultrasonic operation system with a control action of locking an output frequency on to the resonance frequency of an ultrasonic transducer so as to drive the ultrasonic transducer at the resonance frequency.

US 2007/0152081 A1 teaches a micro-spray system resonance frequency modulation method and a device for adjusting resonance frequency in a real-time manner.

US 6,978,779 B2 discloses a nebulizer that determines the pressure and flow direction of a receiving gas for atomized liquid.

Embodiments of the present invention relate to nebulizers. In particular, the present invention relates to use of a variable voltage and frequency driver for a nebulizer having a sealed liquid drug reservoir capable of maintaining a negative internal bias pressure.

A wide variety of procedures have been proposed to deliver a drug to a patient. In some drug delivery procedures the drug is a liquid and is dispensed in the form of fine liquid droplets for inhalation by a patient. A patient may inhale the drug for absorption through lung tissue. Further, the droplets forming the atomized mist may need to be very small to travel through small airways of the lungs. Such a mist may be formed by a nebulizer.

### SUMMARY

Various systems, methods, and devices are described for driving a nebulizer using a driver unit. The nebulizer may include a sealed drug reservoir such that a negative bias pressure may form within the drug reservoir as liquid is drained from it. As the negative bias pressure changes, the resonant frequency of the nebulizer element may change. A driver may be used to drive the nebulizer element and cause it to vibrate. The driver may output a waveform signal of varying frequency and magnitude to the nebulizer such that the nebulizer element vibrates at or near a resonant frequency and the Nebulizer element atomizes liquid at a constant or near constant rate and droplet size.

In some embodiments, a method for determining a resonant frequency of an element of a nebulizer with a negatively biased liquid reservoir is described. The method may include driving a nebulizer using an electrical signal, the electrical signal comprising a current and a voltage. The method may include measuring a phase shift between the voltage and the current of the electrical signal driving the nebulizer. The method may also include, based at least in part on the phase shift of the electrical signal driving the nebulizer, determining, a resonant frequency of the element of the nebulizer.

In some embodiments, the method further comprises based at least in part on the resonant frequency of the nebulizer determined by the driver, determining, a voltage magnitude for the electrical signal. In some embodiments, the negatively biased liquid reservoir causes the resonant frequency of the element of the nebulizer to vary as liquid is drained from the negatively biased liquid reservoir. In some embodiments, the electrical signal driving the nebulizer causes the element of the nebulizer to vibrate and atomize liquid stored in the negatively biased liquid reservoir. In some embodiments, the method further comprises based at least in part on the resonant frequency of the nebulizer determined by the driver, determining, by the driver, a negative bias pressure within the negatively biased liquid reservoir of the nebulizer. In some embodiments, the method further comprises adjusting the frequency of the electrical signal to the nebulizer, wherein a roughly constant phase shift is maintained between the voltage and current of the electrical signal. In some embodiments, the voltage magnitude is determined using a stored set of values, and the stored set of values vary depending on a liquid in the negatively biased liquid reservoir.

In some embodiments, a device for driving an element of a nebulizer is present. The device may include an amplifier, configured to generate an output waveform signal, the output waveform signal comprising an output current and an output voltage, wherein the output waveform signal drives the element of the nebulizer at the output frequency. The device may include a phase shift detector, configured to determine the phase shift between the output current and the output voltage of the output waveform signal. The device may include a resonant frequency tracker, configured to generate a waveform signal of a variable frequency input to the amplifier, wherein the variable frequency is adjusted based on the phase shift of the output waveform signal determined by the phase shift detector module. The device may include a voltage profile, configured to adjust the output voltage of the output waveform signal output by the amplifier based on the frequency of the waveform signal generated by the resonant frequency tracker.

In some embodiments, a system for atomizing liquid stored in a negatively bias pressured liquid reservoir may be present. The system may include a nebulizer, comprising an element and the negatively bias pressured liquid reservoir. The element may be configured to vibrate to atomize liquid drained from the negatively bias pressured liquid reservoir. A negative bias pressure of the negatively bias pressured reservoir may change as liquid stored in the negatively bias pressured liquid reservoir is drained. The negatively bias pressured reservoir may be sealed such that air from the external environment substantially does not enter the negatively bias pressured reservoir as liquid stored in the negatively bias pressured liquid reservoir is drained. The system may include a driver. The driver may include a phase shift detector, configured to determine the phase shift between a current of an output waveform signal and a voltage of the output waveform signal. The driver may include a resonant frequency tracker, configured to generate an output waveform that adjusts the frequency of the output waveform signal, wherein the frequency is adjusted based on the phase shift determined by the phase shift detector module. The driver may include a voltage profile, configured to adjust a voltage of the output waveform signal based on the frequency of the output waveform generated by the resonant frequency tracker.

In some embodiments, a method for aerosolizing a liquid is present. The method may include sealing the liquid within a reservoir. The method may also include generating an output waveform signal and vibrating a nebulizer element to aerosolize the liquid. A negative pressure may be produced within the reservoir as the liquid is aerosolized. The output waveform signal may cause the nebulizer element to vibrate. The method may include determining a phase shift between a current of the output waveform signal and a voltage of the output waveform signal. The method may include adjusting a frequency of the output waveform signal at least partially based on the phase shift. Further, the method may include adjusting the voltage of the output waveform signal at least partially based on the frequency of the output waveform signal.

### BRIEF DESCRIPTION OF THE DRAWINGS

A further understanding of the nature and advantages of the present invention may be realized by reference to the following drawings. In the appended figures, similar components or features may have the same reference label. Further, various components of the same type may be distinguished by following the reference label by a second label that distinguishes among the similar components. If only the first reference label is used in the specification, the description is applicable to any one of the similar components having the same first reference label irrespective of the second reference label.
FIG. 1A illustrates a simplified embodiment of a nebulizer.
FIG. 1B illustrates a simplified embodiment of a nebulizer with a driver unit.
FIG. 1C illustrates a simplified embodiment of a handheld nebulizer with an integrated driver unit.
FIG. 1D illustrates a nebulizer integrated with a ventilator.
FIG. 2 illustrates a simplified embodiment of a driver coupled with a nebulizer.
FIG. 3 illustrates a method of driving a nebulizer with a driver.
FIG. 4 illustrates a method of initially determining a resonant frequency of a nebulizer element.

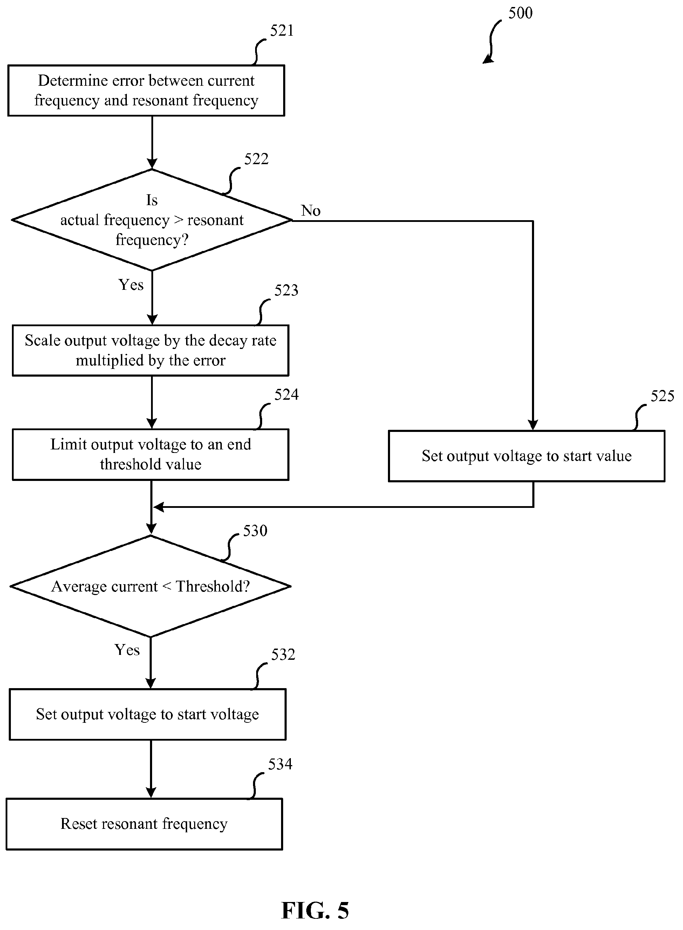

FIG. 5 illustrates a simplified method of adjusting the frequency output by a driver using a resonant frequency tracker to maintain the nebulizer element vibrating at its current resonant frequency

### DETAILED DESCRIPTION OF THE INVENTION

Devices, systems, and methods are described for the implementation of a novel architecture for driving a nebulizer. The invention provides various ways for driving nebulizers at the nebulizers' resonance frequencies, particularly nebulizers with sealed drug reservoirs capable of developing a negative bias pressure (meaning the pressure within the reservoir is less than the pressure outside of the reservoir) as liquid is evacuated from the drug reservoir.

By creating a negative bias pressure within the drug reservoir on a nebulizer the efficiency of a nebulizer may be increased, thus allowing it to achieve higher liquid flow rates, with smaller and more consistent droplet sizes, than in comparable conditions without a negative bias pressure. Such a negative bias pressure may be created by sealing the drug reservoir. As the liquid drug is drained from the drug reservoir (with little to no air entering to replace the drug's volume), a negative bias pressure may be created. While the negative bias pressure may assist in maintaining consistently sized droplets of mist, as the negative bias pressure decreases in pressure, the flow rate of liquid from the nebulizer may increase.

An increased flow rate caused by a negative bias pressure may lead to the wrong dose of a medicine being delivered to a patient and/or the generation of improper droplet sizes. Such improper droplet sizes may alter how the droplets are absorbed into the human body. For example, if a patient inhales droplets that are too large, the droplets may not propagate into the deep lung tissue of the patient, but rather, the droplets may gather in the patient's larger airways. This may prevent proper absorption of the droplets by the patient.

The droplets may be created from a stored amount of liquid in the drug reservoir by a nebulizer element. The nebulizer element may be an aperture plate containing a number of small holes. When an electrical signal, such as a waveform, is applied to the nebulizer element, the nebulizer element may vibrate at or near the frequency of the waveform received, While vibrating, the nebulizer element may allow an amount of the liquid to pass through the element and form airborne droplets. The nebulizer element may function more efficiently and produce consistent droplet sizes when the nebulizer element is vibrating at or near its resonant frequency.

However, as the negative bias pressure within the drug reservoir changes (e.g., a greater difference between the pressure inside the drug reservoir and the ambient pressure outside of the drug reservoir is formed) the resonant frequency of the nebulizer element may change. In order to maintain the nebulizer element vibrating at its (current) resonant frequency, it may be necessary to change the frequency of waveform used to drive the nebulizer element.

Therefore, if a negative bias pressure is maintained in the drug reservoir, the frequency and magnitude of the waveform used to drive the nebulizer element needs to vary as the negative bias pressure within the drug reservoir changes is order to maintain efficient operation of the nebulizer element, including maintaining consistent dosing of the liquid drug and consistent droplet sizes.

To be clear, a sealed reservoir refers to a reservoir that prevents air from entering the reservoir as liquid is drained from the drug reservoir. It may, however, still be possible for air to enter the sealed drug reservoir through holes in the nebulizer element. The greater the negative bias pressure (that is, the greater the difference between the pressure of the external environment and the pressure within the drug reservoir) the faster air may enter through the nebulizer element.

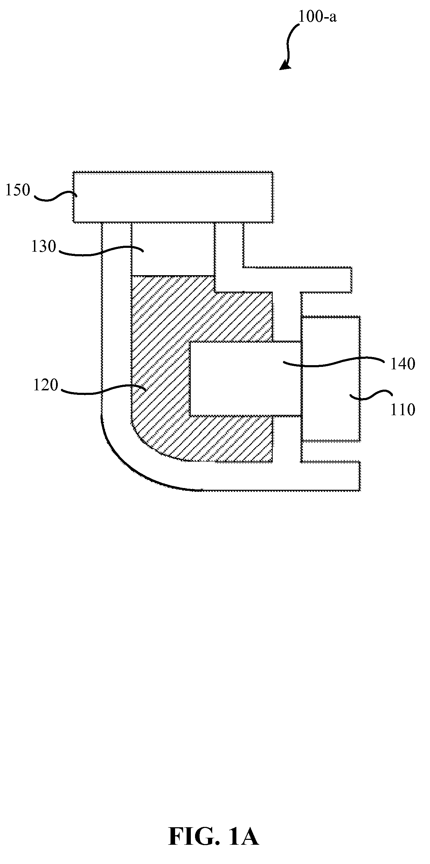

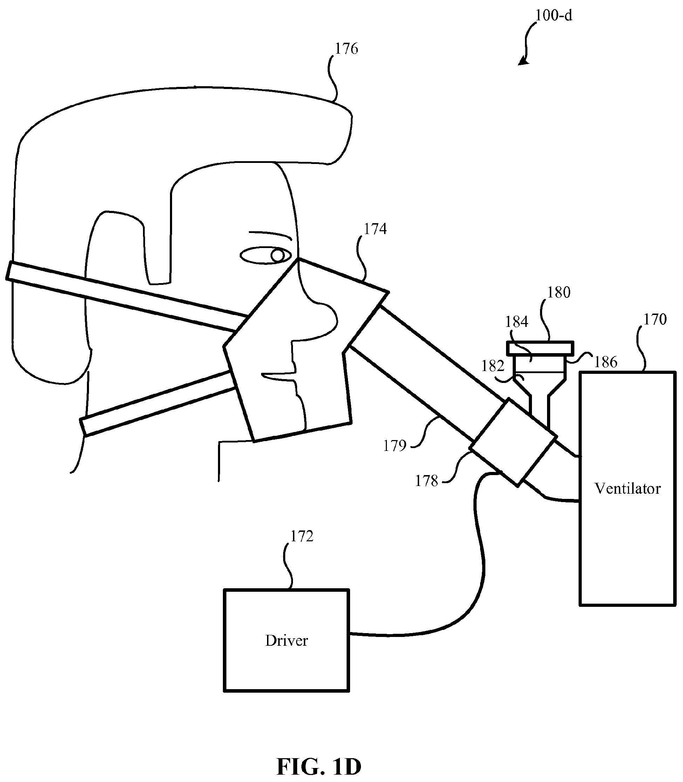

FIG. 1A illustrates an embodiment of a possible nebulizer 100-a. The nebulizer 100-a may includes a nebulizer element 110, a drug reservoir 120, a head space 130, an interface 140, and a cap 150. The nebulizer element 110 may be comprised of a piezoelectric ring that may expand and contract when an electric voltage is applied to the ring. The Nebulizer element 110 may be a vibrating aperture plate. The piezoelectric ring may be attached to a perforated membrane. Such a perforated membrane may have a number of holes passing through it. When an electric voltage is applied to the piezoelectric ring, this may cause the membrane to move and/or flex. Such movement of the membrane, while in contact with a liquid may cause the atomization (alternatively referred to as aerosolization) of the liquid.

A supply of a liquid, commonly a liquid drug, may be held in the drug reservoir 120. As illustrated, a drug reservoir is partially filled with a liquid drug. As the liquid drug is atomized, the amount of liquid drug remaining in the drug reservoir 120 may decrease. Depending on the amount of liquid drug in the drug reservoir 120, only a portion of the reservoir may be filled with liquid drug. The remaining portion of the drug reservoir 120 may be filled with gas, such as air. This space is commonly referred to as head space 130. An interface 140 may serve to transfer amounts of liquid drug between the drug reservoir 120 and the nebulizer element 110.

Nebulizers, and the techniques associated with such nebulizers, are described generally in U.S. Patent Nos. 5,164,740; 5,938,117; 5,586,550; 5,758,637; 6,014,970; 6,085,740; 6,235,177; 6,615,824; 7,322,349.

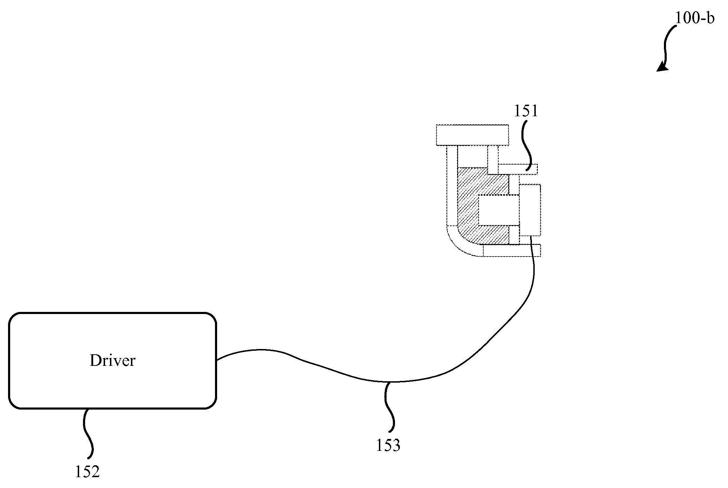

A nebulizer with a sealed drug reservoir may be part of a larger system. The embodiment of FIG. 1B illustrates such a system 100-b. FIG. 1B illustrates a nebulizer 151 with a sealed drug reservoir connected to a driver 152. The sealed nebulizer illustrated in FIG. 1B may be the nebulizer of FIG. 1A, or may represent some other nebulizer. Driver 152 may control the rate and magnitude of vibration of the nebulizer element on nebulizer 151. Driver 152 may be connected to nebulizer element 151 via cable 153. Driver 152 may regulate the voltage and frequency of the signal provided to the nebulizer element of nebulizer 151. The regulation of the voltage and frequency of the signal may be based on the resonant frequency of the nebulizer element of nebulizer 151. Such a signal may vary depending on the magnitude of the negative bias pressure.

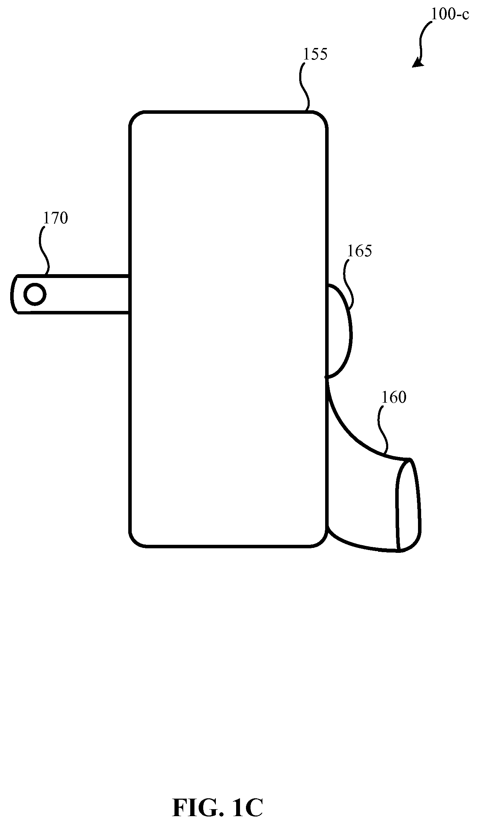

In some other embodiments of nebulizers, a driver may be incorporated into a handheld unit with the nebulizer. Nebulizer 100-c of FIG. 1C illustrates an embodiment of a handheld nebulizer with an integrated driver. Nebulizer 100-c may include a case 155, a mouthpiece 160, a trigger button 165, and an electrical plug 170. Case 155 may contain some or all of the elements found in other embodiments of nebulizers (such as nebulizer 100-a of FIG. 1A) and drivers (such as driver 152 of FIG. 1B). Therefore, contained within case 155 may be a sealed drug reservoir and/or a device capable of generating an electrical signal at a voltage magnitude and frequency to vibrate an element that atomizes liquid stored in the drug reservoir. A person receiving the atomized liquid drug may place her mouth on mouthpiece 160 and breath in. While the person receiving the atomized liquid drug is breathing in, she may press trigger button 165 to trigger the element to begin aerosolizing liquid. In some embodiments, nebulizer 100-c may contain a sensor that detects when the person is breathing in and triggers the element to vibrate without trigger button 165 being necessary.

Nebulizer 100-c may also include an electrical plug 170. Electrical plug 170 may be connected to an electrical outlet to power nebulizer 100-c. Nebulizer 100-c may contain a battery, thereby allowing electrical plug 170 to be connected to an electrical outlet when nebulizer 100-c is not in use by a person, allowing a battery to be charged. Alternatively, in some embodiments of Nebulizer !00-c, electrical plug 170 may need to be connected to an electrical outlet while nebulizer 100-c is in use by a person. In some embodiments, nebulizer 100-c may use replaceable batteries as its power source.

In some embodiments, a nebulizer may operate in conjunction with a ventilator. System 100-d illustrates a Nebulizer 178 that supplies atomized liquid drug to a person 176 via a ventilator 170. Ventilator 170 may supply air suitable for breathing to person 176. Ventilator 170 may assist person 176 in breathing by forcing air into the lungs of person 176 and then releasing air to mimic breathing. While person 176 is using ventilator 170, it may be necessary to provide person 176 with atomized liquid, such as a liquid drug.

Nebulizer 178 may be connected to a drug reservoir 186 thai is sealed by a cap 180. Drug reservoir 186 may contain an amount of liquid drug 182. This liquid drug may be delivered to nebulizer 178 as liquid drug is atomized by nebulizer 178. As liquid drug is atomized, liquid drug 182 may drain from drug reservoir 186, thereby increasing the volume of headspace 184. Headspace 184 may contain air. Headspace 184 may increase in volume, but may decrease in pressure as liquid drug 182 drains because liquid reservoir 186 allows no or minimal air into headspace 184.

Driver 172, which may represent the same driver as driver 152 of FIG. 1B (or may represent some other driver) may deliver a signal to nebulizer 178. This signal may control the vibration of an element of nebulizer 178. Nebulizer 178 may be attached to a tube 179 used to deliver air and atomized liquid drug to patient 176. Tube 179 may terminate in a mask 174 covering the mouth and/or nose of person 176. The air and atomized liquid drug may then enter the airways of person 176.

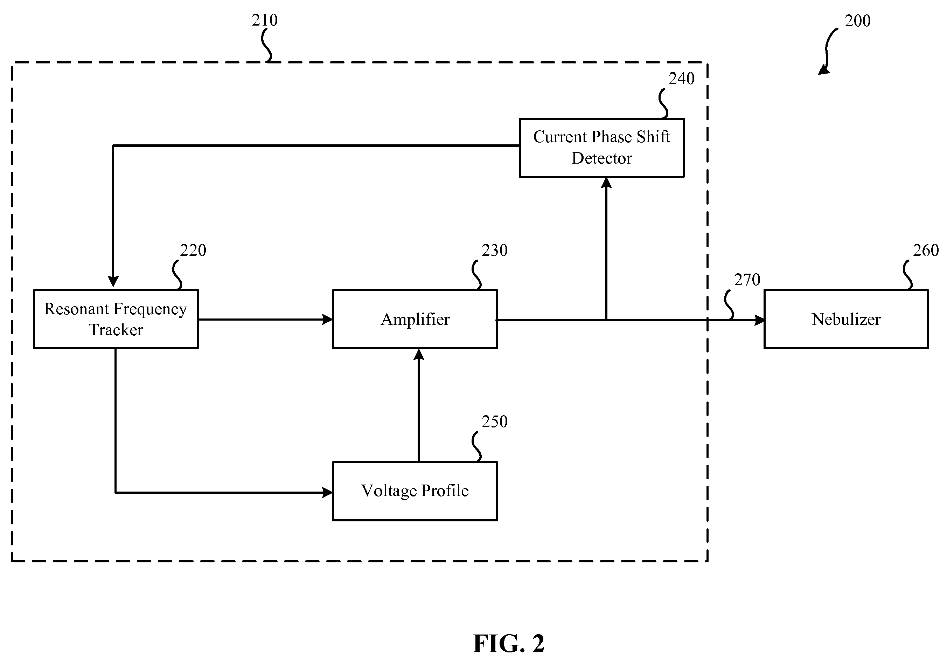

A nebulizer such as those illustrated in FIGs, 1A-1D may be connected with a driver such as illustrated in FIG. 2. FIG. 2 illustrates a simplified block diagram of a nebulizer driver unit 200. The nebulizer 260 may be the Nebulizer 1.00-a of FIG. 1A or it may be some other nebulizer such as those in the referenced applications or FIGs. 1B-1D. The Nebulizer may be connected to the driver via a cable 270. Driver 210 may be driver 151 of FIG. 1B, or may be some other driver. Cable 270 may allow driver 210 to transmit an electrical waveform signal of varying frequency and magnitude (of voltage) through cable 270 to drive nebulizer 260.

Driver 210 may include an amplifier 230, a current phase shift detector 240, a resonant frequency tracker 220, and a voltage profile 250. Based upon the phase shift between the current supplied to nebulizer 260 and the voltage generated by amplifier 230, the nebulizer element's resonant frequency may be determined. From the resonant frequency, the negative bias pressure within the drug reservoir of the nebulizer may be determined, and the frequency and/or magnitude of the electrical waveform signal driving Nebulizer 260 may be adjusted.

The determination of the resonant frequency may be accomplished using current phase shift detector 240. Current phase shift detector 240 monitors the phase shift between the phase of the current output by amplifier 230 to nebulizer 260 and the phase of the voltage output by amplifier 230 to nebulizer 260. Based upon the phase shift between the voltage and current observed by current phase shift detector 240, resonant frequency tracker 220 outputs an output waveform to amplifier 230 such that amplifier 230 outputs an electrical waveform signal with constant or near constant phase shift between the voltage and current of the electrical waveform signal driving the element of nebulizer 260.

As liquid is atomized and the bias pressure in the drug reservoir changes, the resonant frequency may change. Further, factors besides the bias pressure within the sealed drug reservoir of the nebulizer 260 may change the nebulizer element's resonant frequency. For example, the temperature of the nebulizer element, excess liquid on the nebulizer element, and/or damage to the nebulizer element may cause a variation in the nebulizer element's resonant frequency. However, it may be generally accepted that during operation, changes in the nebulizer element's resonant frequency is generally due to variations in the bias pressure within the drug reservoir of the nebulizer.

The resonant frequency and/or the measured change in resonant frequency may be transmitted to voltage profile 250 by resonant frequency tracker 220. Voltage profile 250 may be used to determine the proper magnitude of voltage to apply to the nebulizer element at a particular resonant frequency to maintain consistent droplet size and dosing of the atomized liquid. In some embodiments, voltage profile 250 may include a table of empirically gathered data. In such embodiments, the resonant frequency may be located in the table, with a corresponding analog or digital signal being output to amplifier 230 that specifies the appropriate magnitude of voltage amplifier 230 should output. For example, a table may include a predetermined voltage magnitude that may be communicated to amplifier 230 when a particular resonant frequency is measured by resonant frequency tracker module 220. Voltage profile 250 may also be expressed as a graph of values, with the x-axis being frequency of the waveform generated by resonant frequency tracker 220, and the y-axis representing the appropriate voltage magnitude to be supplied to amplifier 230 such that amplifier 230 outputs an electrical signal of correct magnitude.

A rough description of one set of possible values for voltage profile 250 is that as the resonant frequency of the nebulizer element increases, the desired amplitude of the electrical signal output to the will decrease. At a certain threshold, as the resonant frequency continues to increase, the voltage will be held by voltage profile 250 at a minimum level. In some embodiments of voltage profile 250, the signal output to amplifier 230 are determined based on a calculation using the resonant frequency supplied by resonant frequency tracker 220.

The voltage profile may need to be modified or adjusted to accommodate the characteristics (such as surface tension) of different liquids within the drug reservoir of the nebulizer, In some embodiments, a liquid drug, such as Amikasin, is used. In other embodiments, a different liquid drug or liquid is used. In some embodiments, the voltage profiles necessary for a number of liquids or liquid drugs may be similar enough that only one voltage profile needs to be used for multiple liquids or liquid drugs, Modifying or replacing voltage profile 250 may involve selecting a different liquid via a user interface on driver 210 or loading different software, firmware, and/or hardware into driver 210.

Resonant frequency tracker 220 may transmit a waveform at or near the nebulizer element's current determined resonant frequency to amplifier 230. Voltage profile 250 may transmit a signal indicating the desired voltage amplitude to be output by amplifier 230 to amplifier 230. This signal from voltage profile 230 may serve to control the gain of the amplifier 230. Based upon the input waveform from resonant frequency tracker 220 and the desired voltage amplitude received from voltage profile 250, amplifier 230 generates an output electrical signal that may be used to drive an aperture of the nebulizer. Amplifier 230 may be a variable gain linear power amplifier. In some embodiments, a fixed gain power amplifier may be used in conjunction with a variable gain amplifier or a potentiometer. Further, various other amplifiers or amplifier based circuits may be used to generate the output electrical signal to drive nebulizer 260.

Current phase shift detector 240 may create a feedback loop to resonant frequency tracker 220. Current phase shift detector 240 may determine the phase shift of the current being output from the amplifier 230. Such a phase shift may be transmitted to resonant frequency tracker 220, thereby allowing resonant frequency tracker 220 to either maintain the same frequency signal (if the phase has not shifted), increase the frequency, or decrease the frequency of the output signal in response to the resonant frequency of the nebulizer element changing as the bias pressure within the seal drug reservoir changes. Feedback trough current phase shift detector 240 may allow driver 210 to periodically of continually adjust the magnitude and frequency of the electrical signal output to the nebulizer element while liquid is being atomized. This may allow for any change in the bias pressure in the liquid reservoir to be continually adjusted for by the driver.

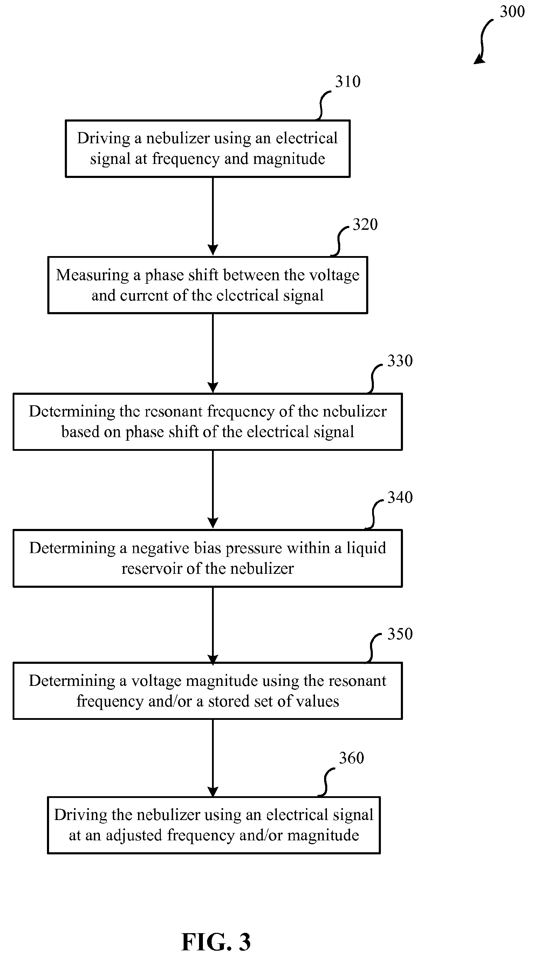

A driver, such as driver 210 of FIG. 2, may drive a nebulizer element according to a method, such as method 300 of FIG. 3. Alternatively, method 300 may be performed using some other driver. Method 300 may employ various different nebulizers, such as the nebulizers of FiGs. 1A-1D, and FIG. 2. At block 310, the driver may drive an element (also referred to as an aperture) of a nebulizer with an electrical signal. This electrical signal may be a waveform at a particular frequency and magnitude.

At block 320, the phase shift between the voltage of the electrical signal output to the nebulizer and the current of the electrical signal may be measured. Using this phase shift, at block 330, the resonant frequency of the nebulizer element may be determined. As previously noted, this resonant frequency may shift as the negative bias pressure within the liquid reservoir of the nebulizer changes. From the resonant frequency, the bias pressure within the liquid reservoir may be determined at block 340. In some embodiments, the negative bias pressure is not determined.

At block 350, the magnitude of the voltage of the electrical signal used to drive the nebulizer element may be determined. The magnitude may be determined using the resonant frequency determined at block 330 and/or the negative bias pressure determined at block 340. The resonant frequency and/or the negative bias pressure may be used to consult a table of values. This table of values may specify the appropriate magnitude of voltage to be used for the electrical signal driving the nebulizer element. Alternatively, the resonant frequency and/or the negative bias pressure may be used to calculate the appropriate voltage magnitude to drive the nebulizer element. The appropriate magnitude may correspond to a magnitude that maintains a constant dosage rate and droplet size of the liquid being dispensed from the nebulizer. The calculations or table may vary depending on the properties of the liquid being dispensed.

At block 360, the electrical waveform signal driving the nebulizer element may be adjusted according to the frequency determined at block 330 and/or the magnitude determined at block 350. If the resonant frequency of the nebulizer element has not changed, the frequency and/or the magnitude of the electrical signal driving the nebulizer element may not change. Method 300 may repeat as long as the nebulizer element is being driven by the driver.

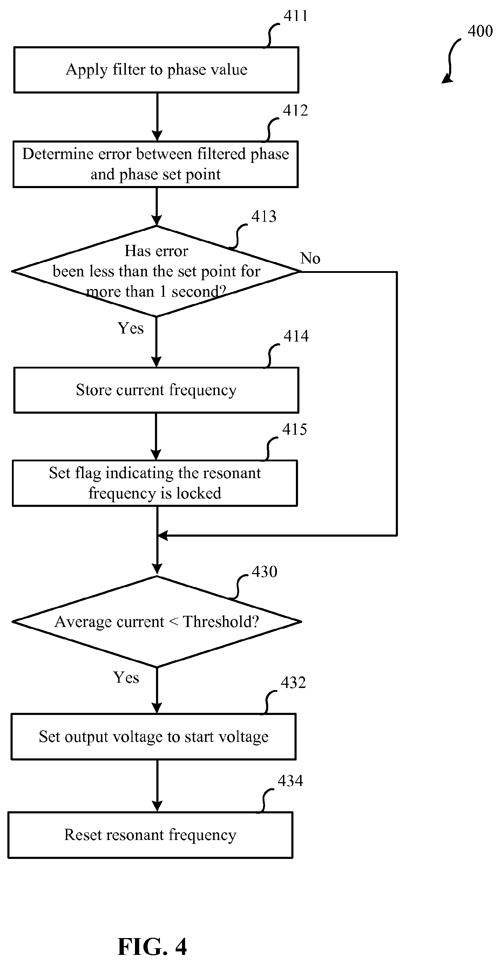

A resonant frequency tracker, such as resonant frequency tracker 220 of FIG. 2, may follow various methods to determine and maintain an output at or near the resonant frequency of a nebulizer element, such as nebulizer element 260 of FIG. 2. FIG. 4 illustrates a simplified flowchart of a decay profile 400 for initially determining the resonant frequency of the nebulizer element and adjusting the output electrical signal driving the nebulizer element based on the phase shift between the voltage and current of the electrical signal driving the nebulizer element defected by the current phase shift detector. Method 300 of FIG. 3 may be implemented using resonant frequency tracker 220 of FIG. 2, or may be implemented using some other resonant frequency tracker, be it implemented in software, firmware, and/or hardware.

If the resonant frequency has not been determined or "looked on" to by a resonant frequency tracker, a resonant frequency tracker may conduct method 400. The resonant frequency tracker may not have locked on to the resonant frequency if, for example, the driver has just been turned on or activated, a new nebulizer is attached to the driver unit, the nebulizer element has been interfered with, or the nebulizer element has been damaged.

At block 411, the resonant frequency, tracker may apply an infinite impulse response filter ("IIR filter"'), to the phase signal received from the current phase shift detector. The IIR filter may be implemented using analog and/or digital components. From this, a filtered phase value may be obtained.

Using the filter phase value, the error between the filtered phase and desired phase setpoint may be determined at block 412. The desired phase set point may indicate the phase necessary to cause the nebulizer element to vibrate at a resonant frequency. This determined error value may then be used to determine if the error has been a smaller value than the set point for greater than a second at block 413. In some embodiments, a different length of time is used.

If the error has been less than the set point for more than one second, the current frequency of the signal output to the nebulizer is stored at block 414. Further, a flag may be set to indicate that the resonant frequency has been locked on to by the resonant frequency tracker at block 415. Returning to block 413, if the error has not been less than the set point for more than one second, the process proceeds to block 430.

At block 430, if the average current is less than some threshold current-value, the output voltage may be set to a start voltage at block 432. At block 434, the resonant frequency determined by the resonant frequency tracker may be reset to an initial value. If the average current is not less than a threshold current value, blocks 432 and 434 may not be performed. Method 400 may repeat until the flag indicating the resonant frequency of the nebulizer element has been locked on to.

Once the resonant frequency has been determined and locked, which may involve the resonant frequency flag of block 414 being set, a second method may be followed. Method 500 represents a method for adjusting the frequency using a resonant frequency tracker to maintain the nebulizer element vibrating at its current resonant frequency. The error between the current frequency and resonant frequency may be determined at block 521. From this, an error value may be obtained.

A determination of whether the actual frequency of the signal being generated by the resonant frequency tracker is greater than the resonant frequency of the nebulizer element may be made at block 522. If yes, at block 523, the output voltage may be scaled by a decay rate multiplied by the error rate determined at block 521, and the output voltage may be limited to the end voltage at block 524. This may prevent the output voltage from exceeding some maximum and/or minimum threshold value. Next, the process proceeds to block 530. If the actual frequency is not determined to be greater than the resonant frequency at block 522, the output voltage is set to a start voltage at block 525, and the method proceeds to block 530.

At block 530, a determination is made if the current is less than a threshold current value. If so, the output voltage is set to the start voltage at block 532 and the resonant frequency is reset at block 534.

While a wide variety of drugs, liquids, liquid drugs, and drugs dissolved in liquid may be aerosolized, the following provides extensive examples of what may be aerosolized. Additional examples are provided in U.S. App. No. 12/341,780. Nearly any anti-gram-negative, anti-gram-positive antibiotic, or combinations thereof may be used. Additionally, antibiotics may comprise those having broad spectrum effectiveness, or mixed spectrum effectiveness. Antifungals, such as polyene materials, in particular, amphotericin B are also suitable for use herein. Examples of anti-gram-negative antibiotics or salts thereof include, but are not limited to, aminoglycosides or salts thereof. Examples of aminoglycosides or salts thereof include gentamicin, amikacin, kanamycin, streptomycin, neomycin, netilmicin, paramecin, tobramycin, salts thereof, and combinations thereof. For instance, gentamicin sulfate is the sulfate salt, or a mixture of such salts, of the antibiotic substances produced by the growth of Micromonospora purpurea. Gentamicin sulfate, USP, may be obtained from Fujian Fukang Pharmaceutical Co., LTD, Fuzhou, China. Amikacin is typically supplied as a sulfate salt, and can be obtained, for example, from Bristol-Myers Squibb. Amikacin may include related substances such as kanamicin.

Examples of anti-gram-positive antibiotics or salts thereof include, but are not limited to, macrolides or salts thereof. Examples of macrolides or salts thereof include, but are not limited to, vancomycin, erythromycin, clarithromycin, azithromycin, salts thereof, and combinations thereof. For instance, vancomycin hydrochloride is a hydrochloride salt of vancomycin, an antibiotic produced by certain strains of Amycolatopsis orientalis, previously designated Streptomyces orientalis. Vancomycin hydrochloride is a mixture of related substances consisting principally of the monohydrochloride of vancomycin B. Like all glycopeptide antibiotics, vancomycin hydrochloride contains a central core heptapeptide, Vancomycin hydrochloride, USP, may be obtained from Alpharma, Copenhagen, Denmark,

In some embodiments, the composition comprises an antibiotic and one or more additional active agents. The Additional active agent described herein includes an agent, drug, or compound, which provides some pharmacologic, often beneficial, effect. This includes foods, food supplements, nutrients, drugs, vaccines, vitamins, and other beneficial agents. As used herein, the terms further include any physiologically or pharmacologically active substance that produces a localized or systemic effect in a patient An active agent for incorporation in the pharmaceutical formulation described herein may be an inorganic or an organic compound, including, without limitation, drugs which act on: the peripheral nerves, adrenergic receptors, cholinergic receptors, the skeletal muscles, the cardiovascular system, smooth muscles, the blood circulatory system, synoptic sites, neuroeffector junctional sites, endocrine and hormone systems, the immunological system, the reproductive system, the skeletal system, autacoid systems, the alimentary and excretory systems, the histamine system, and the central nervous system.

Examples of additional active agents include, but are not limited to, anti-inflammatory agents, bronchodilators, and combinations thereof.

Examples of bronchodilators include, but are not limited to, .beta.-agonists, anti-muscarinic agents, steroids, and combinations thereof. For instance, the steroid may comprise albuterol, such as albuterol sulfate.

Active agents may comprise, for example, hypnotics and sedatives, psychic energizers, tranquilizers, respiratory drugs, anticonvulsants, muscle relaxants, antiparkinson agents (dopamine antagnonists), analgesic, anti-inflammatories, antianxiety drugs (anxiolytics), appetite suppressants, antimigraine agents, muscle contractants, additional anti-infectives (antivirals, antifungals, vaccines) antiarthritics, antimalarials, antiemetics, anepileptics, cytokines, growth factors, anti-cancer agents, antithrombotic agents, antihypertensives, cardiovascular drugs, antiarrhythmics, antioxicants, anti-asthma agents, hormonal agents including contraceptives, sympathomimetics, diuretics, lipid regulating agents, antiandrogenic agents, antiparasitics, anticoagulants, neopisstics, antineoplastics, hypoglycemics, nutritional agents and supplements, growth supplements, antienteritis agents, vaccines, antibodies, diagnostic agents, and contrasting agents. The active agent, when administered by inhalation, may act locally or systemically.

The active agent may fall into one of a number of structural classes, including but not limited to small molecules, peptides, polypeptides, proteins, polysaccharides, steroids, proteins capable of eliciting physiological effects, nucleotides, oligonucleotides, polynucleotides, fats, electrolytes, and the like.

Examples of active agents suitable for use in this invention include but are not limited to one or more of calcitonin, amphotericin B, erythropoietin (EPO), Factor VIII, Factor IX, ceredase, cerezyme, cyclosporin, granulocyte colony stimulating factor (GCSF), thrombopoietin (TPO), alpha-1 proteinase inhibitor, elcatonin, granulocyte macrophage colony stimulating factor (GMCSF), growth hormone, human growth hormone (HGH), growth hormone releasing hormone (GHRH), heparin, low molecular weight heparin (LMWH), interferon alpha, interferon beta, interferon gamma, interleukin-1 receptor, interleukin-2, interleukin-1 receptor antagonist, interleukin-3, interleukin-4, interleukin-6, luteinizing hormone releasing hormone (LHRH), factor IX, insulin, pro-insulin, insulin analogues (e.g., mono-acylated insulin as described in U.S. Pat. No. 5,922,675), amylin, C-peptide, somatostatin, somatostatin analogs including octreotide, vasopressin, follicle stimulating hormone (FSH), insulin-like growth factor (IGF), insulintropin, macrophage colony stimulating factor (M-CSF), nerve growth factor (NGF), tissue growth factors, keratinocyte growth factor (KGF), glial growth factor (GGF), tumor necrosis factor (TNF), endothelial growth factors, parathyroid hormone (PTH), glucagon-like peptide thymosin alpha 1, IIb/IIIa inhibitor, alpha-1 antitrypsin, phosphodiesterase (PDE) compounds, VLA-4 inhibitors, bisphosphonates, respiratory syncytial virus antibody, cystic fibrosis transmembrane regulator (CFTR) gene, deoxyreibonuclease (Dnase), bactericidal/permeability increasing protein (BPI), anti-CMV antibody, 1 3-cis retinoic acid, oleandomycin, troleandomycin, roxithromycin, clarithromycin, davercin, azithromycin, flurithromycin, dirithromycin, josamycin, spiromycin, midecamycin, leucomycin, miocamycin, rokitamycin, andazithromycin, and swinolide A; fluoroquinolones such as ciprofloxacin, ofloxacin, levofloxacin, trovafloxacin, alatrofloxacin, moxifloxicin, norfloxacin, enoxacin, grepafloxacin, gatifloxacin, lomefloxacin, sparfloxacin, temafloxacin, pefloxacin, amifloxacin, fleroxacin, tosufloxacin, prulifloxacin, irloxacin, pazufloxacin, clinafloxacin, and sitafloxacin, teicoplanin, rampolanin, mideplanin, colistin, daptomycin, gramicidin, colistimethate, polymixins such as polymixin B, capreomycin, bacitracin, penems; penicillins including penicllinase-sensitive agents like penicillin G, penicillin V, penicillinase-resistant agents like methicillin, oxacillin, cloxacillin, dicloxacillin, floxacillin, nafcillin; gram negative microorganism active agents like ampicillin, amoxicillin, and hetacillin, cillin, and galampicillin; antipseudomonal penicillins like carbenicillin, ticarcillin, azlocillin, mezlocillin, and piperacillin; cephalosporins like cefpodoxime, cefprozil, ceftbuten, ceftizoxime, ceftriaxone, cephalothin, cephapirin, cephalexin, cephradrine, cefoxitin, cefamandole, cefazolin, cephaloridine, cefaclor, cefadroxil, cephaloglycin, cefuroxime, ceforanide, cefotaxime, cefatrizine, cephacetrile, cefepime, cefixime, cefonicid, cefoperazone, cefotetan, cefinetazole, ceftazidime, loracarbef, and moxalactam, monobactams like aztreonam; and carbapenems such as imipenem, meropenem, pentamidine isethiouate, lidocaine, metaproterenol sulfate, beclomethasone diprepionate, triamcinolone acetamide, budesonide acetonide, fluticasone, ipratropium bromide, flunisolide, cromolyn sodium, ergotamine tartrate and where applicable, analogues, agonists, antagonists, inhibitors, and pharmaceutically acceptable salt forms of the above. In reference to peptides and proteins, the invention is intended to encompass synthetic, native, glycosylated, unglycosylated, pegylated forms, and biologically active fragments, derivatives, and analogs thereof.

Active agents for use in the invention further include nucleic acids, as bare nucleic acid molecules, vectors, associated viral particles, plasmid DNA or RNA or other nucleic acid constructions of a type suitable for transfection or transformation of cells, i.e., suitable for gene therapy including antisense. Further, an active agent may comprise live attenuated or killed viruses suitable for use as vaccines. Other useful drugs include those listed within the Physician's Desk Reference (most recent edition).

The amount of antibiotic or other active agent in the pharmaceutical formulation will be that amount necessary to deliver a therapeutically or prophylactically effective amount of the active agent per unit dose to achieve the desired result. In practice, this will vary widely depending upon the particular agent, its activity, the severity of the condition to be treated, the patient population, dosing requirements, and the desired therapeutic effect. The composition will generally contain any where from about 1 wt % to about 99 wt %, such as from about 2 wt % to about 95 wt %, or from about 5 wt % to 85 wt %, of the active agent, and will also depend upon the relative amounts of additives contained in the composition. The compositions of the invention are particularly useful for active agents that are delivered in doses of from 0.001 mg/day to 100 mg/day, such as in doses from 0.01 mg/day to 75 mg/day, or in doses from 0.10 mg/day to 50 mg/day. It is to be understood that more than one active agent may be incorporated into the formulations described herein and that the use of the term "agent" in no way excludes the use of two or more such agents.

Generally, the compositions are free of excessive excipients. In one or more embodiments, the aqueous composition consists essentially of the anti-gram-negative antibiotic, such as amikacin, or gentamicin or both, and/or salts thereof and water.

Further, in one or more embodiments, the aqueous composition is preservative-free. In this regard, the aqueous composition may be methylparaben-free and/or propylparaben-free. Still further, the aqueous composition may be saline-free.

In one or more embodiments, the compositions comprise an anti-infective and an excipient. The compositions may comprise a pharmaceutically acceptable excipient or carrier which may be taken into the lungs with no significant adverse toxicological effects to the subject, and particularly to the lungs of the subject. In addition to the active agent, a pharmaceutical formulation may optionally include one or more pharmaceutical, excipients which are suitable for pulmonary administration. These excipients, if present, are generally present in the composition in amounts sufficient to perform their intended function, such as stability, surface modification, enhancing effectiveness or delivery of the composition or the like. Thus if present, excipient may range from about 0.01 wt % to about 95 wt %, such as from about 0.5 wt % to about 80 wt %, from about 1 wt % to about 60 wt %, Preferably, such excipients will, in part, serve to further improve the features of the active agent composition, for example by providing more efficient and reproducible delivery of the active agent and/or facilitating manufacturing. One or more excipients may also be provided to serve as bulking agents when it is desired to reduce the concentration of active agent in the formulation.

For instance, the compositions may include one or more osmolality adjuster, such as sodium chloride. For instance, sodium chloride may be added to solutions of vancomycin hydrochloride to adjust the osmolality of the solution. In one or more embodiments, an aqueous composition consists essentially of the anti-gram-positive antibiotic, such as vancomycin hydrochloride, the osmolality adjuster, and water.

Pharmaceutical excipients and additives useful in the present pharmaceutical formulation include but are not limited to amino acids, peptides, proteins, non-biological polymer, biological polymer, carbohydrates, such as sugars, derivatized sugars such as alditols, aldonic acids, esterified sugars, and sugar polymers, which may be present singly or in combination.

Exemplary protein excipients include albumins such as human serum albumin (HSA), recombinant human albumin (rHA), gelatin, casein, hemoglobin, and the like. Suitably amino acids (outside of the dileucyl-peptides of the invention), which may also function in a buffering capacity, include alanine, glycine, arginine, betaine, histidine, glutamic acid, aspartic acid, cysteine, lysine, leucine, isoleucine, valine, methionine, phenylalanine, aspartame, tyrosine, tryptophan, and the like. Referred are amino acids and polypeptides that function as dispersing agents. Amino acids falling into this category include hydrophobic amino acids such as leucine, valine, isoleucine, tryptophan, alanine, methionine, phenylalanine, tyrosine, histidine, and proline.

Carbohydrate excipients suitable for use in the invention include, for example, monosaccharides such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and the like; and alditols, such as mannitol, xylitol, maltitol, lactitol, xylitol sorbitol (glucitol), pyranosyl sorbitol, myoinositol and the like.

The pharmaceutical formulation may also comprise a bluffer or a pH adjusting agent, typically a salt prepared from an organic acid or base. Representative buffers comprise organic acid salts of citric acid, ascorbic acid, gluconic acid, carbonic acid, tartaric acid, succinic acid, acetic acid, or phthalic acid, Tris, tromethamine hydrochloride, or phosphate buffers.

The pharmaceutical formulation may also include polymeric excipients/additives, e.g., polyvinylpyrrolidones, celluloses and derivatized celluloses such as hydroxymethylcellulose, hydroxyethylcellulose, and hydroxypropylmethylcellulose, Ficolls (a polymeric sugar), hydroxyethylstarch, dextrates (e.g., cyclodextrins, such as 2-hydroxypropyl-.beta.-cyclodextrin and sulfobutylether-.beta.-cyclodextrin), polyethylene glycols, and pectin.

The pharmaceutical formulation may further include flavoring agents, taste-masking agents, inorganic salts (for example sodium chloride), antimicrobial agents (for example benzalkonium chloride), sweeteners, antioxidants, antistatic agents, surfactants (for example polysorbates such as "TWEEN 20" and "TWEEN 80"), sorbitan esters, lipids (for example phospholipids such as lecithin and other phosphatidylcholines, phosphatidylethanolamines), fatty acids and fatty esters, steroids (for example cholesterol), and chelating agents (for example EDTA, zinc and other such suitable cations). Other pharmaceutical excipients and/or additives suitable for use in the compositions according to the invention are listed in "Remington: The Science & Practice of Pharmacy", 19.sup.th ed., Williams & Williams, (1995), and in the "Physician's Desk Reference", 52.sup.nd ed., Medical Economics, Montvale, N.J. (1998).

It should be noted that the methods, systems, and devices discussed above are intended merely to be examples. It must be stressed that various embodiments may omit, substitute, or add various procedures or components as appropriate. For instance, it should be appreciated that, in alternative embodiments, the methods may be performed in an order different from that described, and that various steps may be added, omitted, or combined. Also, features described with respect to certain embodiments may be combined in various other embodiments. Different aspects and elements of the embodiments may be combined in a similar manner. Also, it should be emphasized that technology evolves and, thus, many of the elements are examples and should not be interpreted to limit the scope of the invention.

Specific details are given in the description to provide a thorough understanding of the embodiments. However, it will be understood by one of ordinary skill in the art that the embodiments may be practiced without these specific details. For example, well-known processes, algorithms, structures, and techniques have been shown without unnecessary detail in order to avoid obscuring the embodiments. This description provides example embodiments only, and is not intended to limit the scope, applicability, or configuration of the invention. Rather, the preceding description of the embodiments will provide those skilled in the art with an enabling description for implementing embodiments of the invention. Various changes may be made in the function and arrangement of elements without departing from the scope of the invention.

Further, the preceding description generally details aerosolizing liquid drugs. However, it should be understood that liquids besides liquid drugs may be aerosolized using similar devices and methods.

Also, it is noted that the embodiments may be described as a process which is depicted as a flow diagram or block diagram. Although each may describe the operations as a sequential process, many of the operations can be performed in parallel or concurrently. In addition, the order of the operations may be rearranged. A process may have additional steps not included in the figure.

## Claims

1. A method for controlling the vibration of an element (110) of a nebulizer (100-a, 100-c, 151, 178, 260) having a sealed negatively biased liquid drug reservoir (120, 186) by an electrical signal delivered by a driver (152, 172, 210), the method comprising:
driving, by the driver (152, 172, 210), the element (110) of the nebulizer (100-a, 100-c, 151, 178, 260) using the electrical signal, which causes the element (110) of the nebulizer (100-a, 100-c, 151, 178, 260) to vibrate and atomize liquid stored in the negatively biased liquid reservoir (120, 186), the electrical signal comprising a current and a voltage;
**characterized by**
measuring, by a phase shift detector (240) of the driver (152, 172, 210), a phase shift between the voltage and the current of the electrical signal driving the nebulizer (100-a, 100-c, 151, 178, 260);
based at least in part on the phase shift of the electrical signal driving the element (110) of the nebulizer (100-a, 100-c, 151, 178, 260) measured by the phase shift detector (240), determining, by a resonant frequency tracker (220) of the driver (152, 172, 210), a resonant frequency of the element (110) of the nebulizer (100-a, 100-c, 151, 178, 260)
adjusting, by the driver (152, 172, 210), the frequency of the electrical signal to the nebulizer (100-a, 100-c, 151, 178, 260), wherein a near constant phase shift is maintained between the voltage and current of the electrical signal and the resonant frequency of the element of the nebulizer changes as liquid is atomized and the negative bias pressure within the sealed drug reservoir (120, 186) changes; and
based at least in part on the resonant frequency of the nebulizer (100-a, 100-c, 151, 178, 260) determined by the resonant frequency tracker (220) of the driver (152, 172, 210), determining, using a voltage profile (250) of the driver (152, 172, 210), a voltage magnitude for the electrical signal at a particular resonant frequency to maintain consistent droplet size and dosing of the atomized liquid.

2. The method of claim 1, wherein the negatively biased liquid reservoir (120, 186) causes the resonant frequency of the element (110) of the nebulizer (100-a, 100-c, 151, 178, 260) to vary as liquid is drained from the negatively biased liquid reservoir (120, 186).

3. The method of claim 1, further comprising, based at least in part on the resonant frequency of the nebulizer (100-a, 100-c, 151, 178, 260) determined by the driver (152, 172, 210), determining, by the driver (152, 172, 210), a negative bias pressure within the negatively biased liquid reservoir (120, 186) of the nebulizer (100-a, 100-c, 151, 178, 260).

4. The method of claim 1, wherein the voltage magnitude is determined using a stored set of values, and the stored set of values vary depending on a liquid in the negatively biased liquid reservoir (120, 186).

5. A device for driving an element (110) of a nebulizer (100-a, 100-c, 151, 178, 260) according to the method of any of claims 1 to 4, the device comprising:
an amplifier (230), configured to generate an output waveform signal, the output waveform signal comprising an output frequency, an output current, and an output voltage, wherein the output waveform signal drives the element (110) of the nebulizer (100-a, 100-c, 151, 178, 260) at the output frequency;
a phase shift detector (240), configured to determine a phase shift between the output current and the output voltage of the output waveform signal;
a resonant frequency tracker (220), configured to generate a waveform signal of a variable frequency that is input to the amplifier (230), wherein:
the waveform signal controls the output frequency,
the variable frequency is adjusted based on the phase shift of the output waveform signal determined by the phase shift detector module; and
a voltage profile (250), configured to adjust the output voltage of the output waveform signal output by the amplifier (230) based on the frequency of the waveform signal generated by the resonant frequency tracker (220).

6. The device of claim 5, wherein the nebulizer (100-a, 100-c, 151, 178, 260) has a negatively biased liquid reservoir (120, 186) that causes the resonant frequency of the element (110) of the nebulizer (100-a, 100-c, 151, 178, 260) to vary as liquid is drained from the negatively biased liquid reservoir (120, 186).

7. The driver device of claim 5, wherein the output voltage of the amplifier (230) is determined using a stored set of values, and the stored set of values varies depending on a liquid stored in the negatively biased liquid reservoir (120, 186).

8. The driver device of claim 6, wherein the liquid stored in the negatively biased liquid reservoir (120, 186) is a drug.

9. The driver device of claim 5, wherein the driver device is coupled with the nebulizer (100-c) in a handheld unit.

10. A system for controlling the vibration of an element of a nebulizer for atomizing liquid, the system comprising:
a liquid reservoir (120, 186) that is adapted to hold a liquid that is to be atomized;
a nebulizer (100-a, 100-c, 151, 178, 260), comprising an element (110) having a plurality of apertures, wherein:
the element (110) is configured to vibrate to atomize liquid drained from the liquid reservoir (120, 186) wherein the element is driven by an electrical output waveform signal;
a negative bias pressure of the liquid reservoir (120, 186) changes as liquid stored in the liquid reservoir is drained; and
the liquid reservoir (120, 186) is sealed such that air from the ambient environment substantially does not enter the liquid reservoir (120, 186) as liquid stored in the liquid reservoir (120, 186) is drained; and
a driver (152, 172, 210),
**characterized in that** the driver (152, 172, 210) comprises:
an amplifier (230) for outputting the electrical output waveform signal,
a phase shift detector (240), configured to determine a phase shift between a current of the output waveform signal and a voltage of the output waveform signal;
a resonant frequency tracker (220), configured to generate a waveform being output to the amplifier (230), the waveform adjusting the frequency of the output waveform signal, wherein the frequency is adjusted based on the phase shift determined by the phase shift detector (240) such that the amplifier (230) outputs the output waveform signal with a near constant phase shift between the voltage and current of the output waveform signal and the resonant frequency of the element (110) of the nebulizer (100-a, 100-c, 151, 178, 260) changes as liquid is atomized and the bias pressure in the drug reservoir changes (120, 186); and
a voltage profile (250), configured to adjust the voltage of the output waveform signal based on the frequency of the waveform generated by the resonant frequency tracker (220).

11. The system of claim 10, wherein the nebulizer (178) is configured to be coupled with a ventilator.

12. The system of claim 10, wherein the driver is coupled with the nebulizer (100-c) in a handheld unit.

13. The system of claim 10, wherein the amplifier (230) is configured to generate the output waveform signal using signals from the resonant frequency tracker (220) and the voltage profile (250).

14. The system of claim 10, wherein the output voltage of the amplifier (230) is determined using a stored set of values, and the stored set of values vary depending on the liquid stored in the liquid reservoir (120, 186).

15. The system of claim 14, wherein the liquid is a drug.

## Patentansprüche

1. Verfahren zum Steuern der Vibrationen eines Elements (110) eines Inhalators (100-a, 100-c, 151, 178, 260), der einen dicht geschlossenen, mit einem Unterdruck beaufschlagten Vorratsbehälter für Flüssigmedikamente (120, 186) aufweist, durch ein elektrisches Signal, das von einem Treiber (152, 172, 210) geliefert wird, wobei das Verfahren Folgendes umfasst:
Antreiben des Elements (110) des Inhalators (100-a, 100-c, 151, 178, 260) durch den Treiber (152, 172, 210) unter Verwendung des elektrischen Signals, das das Element (110) des Inhalators (100-a, 100-c, 151, 178, 260) veranlasst zu vibrieren und die Flüssigkeit zu zerstäuben, die in dem mit einem Unterdruck beaufschlagten Flüssigkeitsvorratsbehälter (120, 186) gespeichert ist, wobei das elektrische Signal einen Strom und eine Spannung umfasst;
**gekennzeichnet durch**
Messen einer Phasenverschiebung zwischen der Spannung und dem Strom des elektrischen Signals, das den Inhalator (100-a, 100-c, 151, 178, 260) treibt, **durch** einen Phasenverschiebungsdetektor (240) des Treibers (152, 172, 210);
Bestimmen einer Resonanzfrequenz des Elements (110) des Inhalators (100-a, 100-c, 151, 178, 260) **durch** eine Resonanzfrequenznachführeinheit (220) des Treibers (152, 172, 210), zumindest teilweise auf der Basis der Phasenverschiebung des elektrischen Signals, das das Element (110) des Inhalators (100-a, 100-c, 151, 178, 260) treibt, die **durch** den Phasenverschiebungsdetektor (240) gemessen wurde;
Einstellen der Frequenz des elektrischen Signals an den Inhalator (100-a, 100-c, 151, 178, 260) **durch** den Treiber (152, 172, 210), wobei eine nahezu konstante Phasenverschiebung zwischen der Spannung und dem Strom des elektrischen Signals aufrechterhalten wird und sich die Resonanzfrequenz des Elements des Inhalators ändert, wenn die Flüssigkeit zerstäubt wird, und sich ferner der beaufschlagte Unterdruck in dem dicht geschlossenen Medikamentenvorratsbehälter (120, 186) ändert; und
Bestimmen eines Spannungspegels für das elektrische Signal bei einer bestimmten Resonanzfrequenz unter Verwendung eines Spannungsprofils (250) des Treibers (152, 172, 210), zumindest teilweise auf der Basis der Resonanzfrequenz des Inhalators (100-a, 100-c, 151, 178, 260), die **durch** die Resonanzfrequenznachführeinheit (220) des Treibers (152, 172, 210) bestimmt wurde, um eine konsistente Tropfengröße und Dosierung der zerstäubten Flüssigkeit aufrechtzuerhalten.

2. Verfahren nach Anspruch 1, wobei der mit einem Unterdruck beaufschlagte Flüssigkeitsvorratsbehälter (120, 186) bewirkt, dass sich die Resonanzfrequenz des Elements (110) des Inhalators (100-a, 100-c, 151, 178, 260) verändert, wenn Flüssigkeit aus dem mit einem Unterdruck beaufschlagten Flüssigkeitsvorratsbehälter (120, 186) entleert wird.

3. Verfahren nach Anspruch 1, das ferner das Bestimmen eines beaufschlagten Unterdrucks in dem mit einem Unterdruck beaufschlagten Flüssigkeitsvorratsbehälter (120, 186) des Inhalators (100-a, 100-c, 151, 178, 260) durch den Treiber (152, 172, 210), zumindest teilweise auf der Basis der Resonanzfrequenz des Inhalators (100-a, 100-c, 151, 178, 260), die durch den Treiber (152, 172, 210) bestimmt wurde, umfasst.

4. Verfahren nach Anspruch 1, wobei der Spannungspegel unter Verwendung eines gespeicherten Wertebereichs bestimmt wird und sich der gespeicherte Wertebereich abhängig von einer Flüssigkeit in dem mit einem Unterdruck beaufschlagten Flüssigkeitsvorratsbehälter (120, 186) ändert.

5. Vorrichtung zum Treiben eines Elements (110) eines Inhalators (100-a, 100-c, 151, 178, 260) gemäß dem Verfahren nach einem der Ansprüche 1 bis 4, wobei die Vorrichtung Folgendes umfasst:
einen Verstärker (230), der konfiguriert ist, ein Ausgangswellenformsignal zu erzeugen, wobei das Ausgangswellenformsignal eine Ausgangsfrequenz, einen Ausgangsstrom und eine Ausgangsspannung umfasst, wobei das Ausgangswellenformsignal das Element (110) des Inhalators (100-a, 100-c, 151, 178, 260) mit der Ausgangsfrequenz treibt;
einen Phasenverschiebungsdetektor (240), der konfiguriert ist, eine Phasenverschiebung zwischen dem Ausgangsstrom und der Ausgangsspannung des Ausgangswellenformsignals zu bestimmen;
eine Resonanzfrequenznachführeinheit (220), die konfiguriert ist, ein Wellenformsignal mit einer veränderlichen Frequenz zu erzeugen, das in den Verstärker (230) eingegeben wird, wobei:
das Wellenformsignal die Ausgangsfrequenz steuert,
die veränderliche Frequenz auf der Basis der Phasenverschiebung des Ausgangswellenformsignals eingestellt wird, die durch das Phasenverschiebungsdetektormodul bestimmt wurde; und
ein Spannungsprofil (250), das konfiguriert ist, die Ausgangsspannung des Ausgangswellenformsignalausgangs durch den Verstärker (230) auf der Basis der Frequenz des Wellenformsignals, das durch die Resonanzfrequenznachführeinheit (220) erzeugt wird, einzustellen.

6. Vorrichtung nach Anspruch 5, wobei der Inhalator (100-a, 100-c, 151, 178, 260) einen mit einem Unterdruck beaufschlagten Flüssigkeitsvorratsbehälter (120, 186) aufweist, der verursacht, dass sich die Resonanzfrequenz des Elements (110) des Inhalators (100-a, 100-c, 151, 178, 260) verändert, wenn Flüssigkeit aus dem mit einem Unterdruck beaufschlagten Flüssigkeitsvorratsbehälter (120, 186) entleert wird.

7. Treiber nach Anspruch 5, wobei die Ausgangsspannung des Verstärkers (230) unter Verwendung eines gespeicherten Wertebereichs bestimmt wird und wobei sich der gespeicherte Wertebereich abhängig von einer Flüssigkeit ändert, die in dem mit einem Unterdruck beaufschlagten Flüssigkeitsvorratsbehälter (120, 186) gespeichert ist.

8. Treiber nach Anspruch 6, wobei die in dem mit einem Unterdruck beaufschlagten Flüssigkeitsvorratsbehälter (120, 186) gespeicherte Flüssigkeit ein Medikament ist.

9. Treiber nach Anspruch 5, wobei der Treiber in einer tragbaren Einheit mit dem Inhalator (100-c) verbunden ist.

10. System zum Steuern der Vibrationen eines Elements eines Inhalators zum Zerstäuben einer Flüssigkeit, wobei das System Folgendes umfasst:
einen Flüssigkeitsvorratsbehälter (120, 186), der ausgelegt ist, eine Flüssigkeit zu enthalten, die zerstäubt werden soll;
einen Inhalator (100-a, 100-c, 151, 178, 260), der ein Element (110) umfasst, das mehrere Öffnungen aufweist, wobei:
das Element (110) konfiguriert ist zu vibrieren, um eine Flüssigkeit zu zerstäuben, die aus dem Flüssigkeitsvorratsbehälter (120, 186) entleert wird, wobei das Element von einem elektrischen Ausgangswellenformsignal getrieben wird;
sich ein beaufschlagter Unterdruck des Flüssigkeitsvorratsbehälters (120, 186) ändert, wenn die in dem Flüssigkeitsvorratsbehälter gespeicherte Flüssigkeit entleert wird; und
der Flüssigkeitsvorratsbehälter (120, 186) derart dicht geschlossen ist, dass die Luft aus dem umgebenden Umfeld im Wesentlichen nicht in den Flüssigkeitsvorratsbehälter (120, 186) eindringt, wenn die in dem Flüssigkeitsvorratsbehälter (120, 186) gespeicherte Flüssigkeit entleert wird; und
ein Treiber (152, 172, 210),
**dadurch gekennzeichnet, dass** der Treiber (152, 172, 210) Folgendes umfasst:
einen Verstärker (230) zum Ausgeben des elektrischen Ausgangswellenformsignals,
einen Phasenverschiebungsdetektor (240), der konfiguriert ist, eine Phasenverschiebung zwischen einem Strom des Ausgangswellenformsignals und einer Spannung des Ausgangswellenformsignals zu bestimmen;
eine Resonanzfrequenznachführeinheit (220), die konfiguriert ist, eine Wellenform zu erzeugen, die an den Verstärker (230) ausgegeben wird, wobei die Wellenform die Frequenz des Ausgangswellenformsignals einstellt, wobei die Frequenz auf der Basis der Phasenverschiebung, die durch den Phasenverschiebungsdetektor (240) bestimmt wurde, derart eingestellt wird, dass der Verstärker (230) das Ausgangswellenformsignal mit einer nahezu konstanten Phasenverschiebung zwischen der Spannung und dem Strom des Ausgangswellenformsignals ausgibt und sich die Resonanzfrequenz des Elements (110) des Inhalators (100-a, 100-c, 151, 178, 260) ändert, wenn die Flüssigkeit zerstäubt wird und sich der beaufschlagte Druck in dem Medikamentenvorratsbehälter ändert (120, 186); und
ein Spannungsprofil (250), das konfiguriert ist, auf der Basis der Frequenz der Wellenform, die durch die Resonanzfrequenznachführeinheit (220) erzeugt wird, die Spannung des Ausgangswellenformsignals einzustellen.

11. System nach Anspruch 10, wobei der Inhalator (178) konfiguriert ist, mit einem Ventilator verbunden zu werden.

12. System nach Anspruch 10, wobei der Treiber in einer tragbaren Einheit mit dem Inhalator (100-c) verbunden ist.

13. System nach Anspruch 10, wobei der Verstärker (230) konfiguriert ist, das Ausgangswellenformsignal unter Verwendung der Signale von der Resonanzfrequenznachführeinheit (220) und des Spannungsprofils (250) zu erzeugen.

14. System nach Anspruch 10, wobei die Ausgangsspannung des Verstärkers (230) unter Verwendung eines gespeicherten Wertebereichs bestimmt wird und der gespeicherte Wertebereich sich abhängig von der in dem Flüssigkeitsvorratsbehälter (120, 186) gespeicherten Flüssigkeit ändert.

15. System nach Anspruch 14, wobei die Flüssigkeit ein Medikament ist.

## Revendications

1. Procédé de commande de la vibration d'un élément (110) d'un nébulisateur (100-a, 100-c, 151, 178, 260) par un signal électrique fourni par un dispositif de commande (152, 172, 210), le nébulisateur ayant un réservoir étanche de médicament liquide alimenté avec une dépression (120, 186), le procédé comprenant les étapes de :
commander à l'aide du dispositif de commande (152, 172, 210), l'élément (110) du nébulisateur (100-a, 100-c, 151, 178, 260) utilisant le signal électrique qui provoque la vibration de l'élément (110) du nébulisateur (100-a, 100-c, 151, 178, 260) et la vaporisation du liquide stocké dans le réservoir de liquide alimenté avec une dépression (120, 186), le signal électrique comprenant un courant et une tension ;
**caractérisé par** les étapes de :
mesurer par un détecteur de déphasage (240) du dispositif de commande (152, 172, 210), un déphasage entre la tension et le courant du signal électrique commandant le nébulisateur (100-a, 100-c, 151, 178, 260) ;
en se basant au moins en partie sur le déphasage du signal électrique commandant l'élément (110) du nébulisateur (100-a, 100-c, 151, 178, 260) mesuré par le détecteur de déphasage (240), déterminer, via un traqueur de fréquence de résonance (220) du dispositif de commande (152, 172, 210), une fréquence de résonance de l'élément (110) du nébulisateur (100-a, 100-c, 151, 178, 260)
ajuster, via le dispositif de commande (152, 172, 210), la fréquence du signal électrique fourni au nébulisateur (100-a, 100-c, 151, 178, 260), dans lequel un déphasage presque constant est maintenu entre la tension et le courant du signal électrique et dans lequel la fréquence de résonance de l'élément du nébulisateur change lorsque le liquide est vaporisé et la dépression alimentée à l'intérieur du réservoir étanche de médicament (120, 186) change, et
en se basant sur au moins en partie la fréquence de résonance du nébulisateur (100-a, 100-c, 151, 178, 260) déterminé par le traqueur de fréquence de résonance (220) du dispositif de commande (152, 172, 210), déterminer, en utilisant un profil de tension (250) du dispositif de commande (152, 172, 210), une amplitude de tension pour le signal électrique à une fréquence de résonance particulière pour maintenir une taille de gouttelettes cohérente et réaliser un dosage du liquide vaporisé.

2. Procédé selon la revendication 1, dans lequel le réservoir de liquide alimenté avec une dépression (120, 186) provoque la variation de la fréquence de résonance de l'élément (110) du nébulisateur (100-a, 100-c, 151, 178, 260) lorsque le liquide est drainé à partir du réservoir de liquide alimenté avec une dépression (120, 186).

3. Procédé selon la revendication 1, comprenant en outre, en se basant au moins en partie sur la fréquence de résonance du nébulisateur (100-a, 100-c, 151, 178, 260) déterminé par le dispositif de commande (152, 172, 210), l'étape de déterminer, via le dispositif de commande (152, 172, 210), une dépression alimentée à l'intérieur du réservoir de liquide alimenté avec une dépression (120, 186) du nébulisateur (100-a, 100-c, 151, 178, 260).

4. Procédé selon la revendication 1, dans lequel l'amplitude de tension est déterminée en utilisant un ensemble de données stockées, et dans lequel l'ensemble de valeurs stockées varie en fonction d'un liquide dans le réservoir de liquide alimenté avec une dépression (120, 186).

5. Dispositif pour commander un élément (110) d'un nébulisateur (100-a, 100-c, 151, 178, 260) selon le procédé de l'une quelconque des revendications 1 à 4, le dispositif comprenant :
un amplificateur (230), configuré pour générer un signal de forme d'onde de sortie, le signal de forme d'onde de sortie comprenant une fréquence de sortie, un courant de sortie et une tension de sortie, dans lequel le signal de forme d'onde de sortie commande l'élément (110) du nébulisateur (100-a, 100-c, 151, 178, 260) à la fréquence de sortie ;
un détecteur de déphasage (240), configuré pour déterminer un déphasage entre le courant de sortie et la tension de sortie du signal de forme d'onde de sortie ;
un traqueur de fréquence de résonance (220), configuré pour générer un signal de forme d'onde d'une fréquence variable qui est fourni en entrée d'un amplificateur (230), dans lequel :
le signal de forme d'onde commande la fréquence de sortie,
la fréquence variable est ajustée sur la base du déphasage du signal de forme d'onde de sortie déterminé par le module détecteur de déphasage ; et
un profil de tension (250), configuré pour ajuster la tension de sortie du signal de forme d'onde de sortie fourni par l'amplificateur (230) basé sur la fréquence du signal de forme d'onde produit par le traqueur de fréquence de résonance (220).

6. Dispositif selon la revendication 5, dans lequel le nébulisateur (100-a, 100-c, 151, 178, 260) comprend un réservoir de liquide alimenté avec une dépression (120, 186) qui entraîne la variation de la fréquence de résonance de l'élément (110) du nébulisateur (100-a, 100-c, 151, 178, 260) lorsqu'un liquide est drainé à partir du réservoir de liquide alimenté avec une dépression (120, 186).

7. Dispositif de commande selon la revendication 5, dans lequel la tension de sortie de l'amplificateur (230) est déterminé en utilisant un ensemble de valeurs stocké et dans lequel l'ensemble de valeurs stocké varie en fonction d'un liquide stocké dans le réservoir de liquide alimenté avec une dépression (120, 186).

8. Dispositif de commande selon la revendication 6, dans lequel le liquide stocké dans le réservoir de liquide alimenté avec une dépression (120, 186) est un médicament.

9. Dispositif de commande selon la revendication 5, dans lequel le dispositif de commande est couplé avec le nébulisateur (100-c) dans une unité portable à la main.

10. Système pour commander la vibration d'un élément de nébulisateur pour vaporiser un liquide, le système comprenant :
un réservoir de liquide (120, 186) qui est apté à contenir un liquide qui doit être vaporisé ;
un nébulisateur (100-a, 100-c, 151, 178, 260), comprenant un élément (110) ayant une pluralité d'orifices, dans lequel :
l'élément (110) est configuré pour vibrer pour vaporiser du liquide drainé à partir du réservoir de liquide (120, 186) dans lequel l'élément est commandé par un signal électrique de forme d'onde de sortie ;
une dépression alimentée du réservoir de liquide (120, 186) change lorsque le liquide stocké dans le réservoir de liquide est drainé ; et
le réservoir de liquide (120, 186) est étanche de sorte que l'air de l'environnement ambiant ne pénètre pas de manière substantielle dans le réservoir de liquide (120, 186) lorsque le liquide stocké dans le réservoir de liquide (120, 186) est drainé ; et
un dispositif de commande (152, 172, 210),
**caractérisé en ce que** le dispositif de commande (152, 172, 210) comprend :
un amplificateur (230) pour fournir le signal électrique de forme d'onde de sortie,
un détecteur de déphasage (240), configuré pour déterminer un déphasage entre un courant du signal de forme d'onde de sortie et une tension du signal de forme d'onde de sortie ;
un traqueur de fréquence de résonance (220), configuré pour générer une forme d'onde qui est fourni à l'amplificateur (230), la forme d'onde ajustant la fréquence du signal de forme d'onde de sortie, dans lequel la fréquence est ajustée en se basant sur le déphasage déterminé par le détecteur de déphasage (240) de sorte que l'amplificateur (230) fournit le signal de forme d'onde de sortie avec un déphasage presque constant entre la tension et le courant du signal de forme d'onde de sortie et dans lequel la fréquence de l'élément (110) du nébulisateur (100-a, 100-c, 151, 178, 260) change lorsque le liquide est vaporisé et la dépression alimentée dans le réservoir de médicament change (120, 186) ; et
un profil de tension (250), configuré pour ajuster la tension du signal de forme d'onde de sortie basé sur la fréquence de la forme d'onde générée par le traqueur de fréquence de résonance (220).

11. Système selon la revendication 10, dans lequel le nébulisateur (178) est configuré pour être couplé à un ventilateur.

12. Système selon la revendication 10, dans lequel le circuit de commande est couplé avec le nébulisateur (100-c) dans une unité portable à la main.

13. Système selon la revendication 10, dans lequel l'amplificateur (230) est configuré pour produire le signal de forme d'onde de sortie en utilisant des signaux provenant du traqueur de fréquence de résonance (220) et le profil de tension (250).

14. Système selon la revendication 10, dans lequel la tension de sortie de l'amplificateur (230) est déterminée en utilisant un ensemble de valeurs stocké, et dans lequel l'ensemble des valeurs stocké varie en fonction du liquide stocké dans le réservoir de liquide (120, 186).

15. Système selon la revendication 14, dans lequel le liquide est un médicament.
